(19) Europäisches Patentamt
European Patent Office
Office européen des brevets

(11) **EP 2 968 201 B2**

(12) **NEW EUROPEAN PATENT SPECIFICATION**
After opposition procedure

(45) Date of publication and mention
of the opposition decision:
**18.03.2026   Bulletin 2026/12**

(45) Mention of the grant of the patent:
**28.12.2022   Bulletin 2022/52**

(21) Application number: **14719442.7**

(22) Date of filing: **13.03.2014**

(51) International Patent Classification (IPC):
**A61K 9/70** (2006.01)        **A61K 31/192** (2006.01)
**A61K 47/10** (2017.01)       **A61K 47/32** (2006.01)

(52) Cooperative Patent Classification (CPC):
**A61K 9/7053; A61K 31/192; A61K 47/10;
A61K 47/32**

(86) International application number:
**PCT/US2014/025184**

(87) International publication number:
**WO 2014/159801 (02.10.2014 Gazette 2014/40)**

(54) **ENHANCED DRUG DELIVERY FROM ADHESIVES**

VERBESSERTE ARZNEIMITTELABGABE AUS HAFTSTOFFEN

ADMINISTRATION DE MÉDICAMENT AMÉLIORÉE À PARTIR D'ADHÉSIFS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.03.2013   US 201361778620 P**

(43) Date of publication of application:
**20.01.2016   Bulletin 2016/03**

(60) Divisional application:
**22207478.3 / 4 162 931**

(73) Proprietor: **Avery Dennison Corporation
Mentor, OH 44060 (US)**

(72) Inventors:
• **CARTY, Neal
Mentor, OH 44060 (US)**
• **WIBAUX, Anne Marie
Cleveland Heights, OH (US)**

(74) Representative: **Müller-Boré & Partner
Patentanwälte PartG mbB
Friedenheimer Brücke 21
80639 München (DE)**

(56) References cited:
**EP-A1- 0 764 441        EP-A1- 2 110 125
WO-A1-02/087645     US-B1- 6 280 765**

• **ANONYMOUS: "Issues in Pharmacology,
Pharmacy, Drug Research, and Drug
Innovation", 9 January 2012,
SCHOLARLYEDITIONS, USA**
• **MARTIN SCHULZ: "ADSORPTION PROPERTIES
OF CROSPOVIDONE AND USE
OFCROSPOVIDONE IN DRUG-IN-ADHESIVE
PATCHES", March 2009 (2009-03-01), pages 32 -
40, Retrieved from the Internet <URL:http://www.
diss.fu-berlin.de/diss/servlets/
MCRFileNodeServlet/
FUDISS_derivate_000000005948/
Martin_Schulz_Online-Publikation_Disserta
tion_2009.pdf>**
• **A. PYKA: "Lipophilicity Investigations of
Ibuprofen", JOURNAL OF LIQUID
CHROMATOGRAPHY AND RELATED
TECHNOLOGIES, vol. 32, no. 5, 9 February 2009
(2009-02-09), US, pages 723 - 731, XP055531755,
ISSN: 1082-6076, DOI: 10.1080/
10826070802711220**

Remarks:
The file contains technical information submitted
after the application was filed and not included in this
specification

**EP 2 968 201 B2**

**Description**

The present invention relates to an adhesive composition, to an article adapted to be adhered to biological skin and release at least one active agent to the skin, to a method of releasing at least one active agent to a region of interest on biological skin, and to a method of increasing at least one of (i) extent of release and (ii) rate of release of at least one active agent from an adhesive composition. The present invention is particularly directed to medical adhesives containing one or more pharmaceutical active agents, and more particularly, the present invention relates to improving release and release characteristics of actives from adhesive compositions.

Adhesive compositions are known which release one or more active agents. Although satisfactory in certain respects, some compositions exhibit unpredictable variations in the rate of release of the active(s). In addition, some compositions are not biologically compatible and so should not be left in contact with biological skin or tissue for an extended period of time, such as for example more than 24 hours. EP 2 110 125 A1 relates to a transdermal patch which contains crosslinked PVP as a water absorbing material in the matrix layer.

Accordingly, a need exists for improved adhesive compositions which exhibit predictable release characteristics, are biologically compatible, and which can be readily tailored and utilized in a variety of different applications.

The difficulties and drawbacks associated with previously known compositions are addressed by the present invention. Generally, the present invention relates to improving drug or active agent delivery, rate of delivery, and/or delivery characteristics by incorporation of one or more absorbents in an adhesive composition.

In one aspect, the present invention provides an adhesive composition for use as a medicament wherein the method of releasing at least one active agent to a region of interest on biological skin comprises: providing an article adapted for placement along biological skin, the article defining at least one face; providing an adhesive composition including 30% to 80% of an adhesive component, 0.5% to 45% of at least one absorbent, 0.5% to 10% of at least one active agent, 0.5% to 20% of a vehicle, and 0.5% to 20% of at least one crystallization inhibitor, wherein the at least one active agent exists in a stable amorphous state within the adhesive composition, the adhesive component is a styrene-isoprene hot melt adhesive, the at least one absorbent is carboxymethyl cellulose, the at least one active agent is an anti-rheumatic selected from the group consisting of diclofenac, ibuprofen, piroxicam, ketoprofen, thiocolchicoside, and methotrexate, the at least one crystallization inhibitor is polyvinylpyrrolidone, the vehicle is a polyhydric alcohol, and the various components are homogeneously blended into a single cohesive mass; depositing the adhesive composition onto the face of the article; and applying the article to a region of interest on biological skin, whereby the adhesive composition is directed toward and overlies the region of interest. As described herein, incorporating an absorbent in the adhesive composition containing the active agent, results in improved release and/or release characteristics of the active agent from the composition.

In another aspect, the present invention provides an adhesive composition for use as a medicament wherein the increase of at least one of (i) extent of release and (ii) rate of release of at least one active agent from an adhesive composition including 30% to 80% of an adhesive component, 0.5% to 10% of at least one active agent, 0.5% to 20% of a vehicle, and 0.5% to 20% of at least one crystallization inhibitor, wherein the at least one active agent exists in a stable amorphous state within the adhesive composition, the adhesive component is a styrene-isoprene hot melt adhesive, the at least one active agent is an anti-rheumatic selected from the group consisting of diclofenac, ibuprofen, piroxicam, ketoprofen, thiocolchicoside, and methotrexate, the at least one crystallization inhibitor is polyvinylpyrrolidone, the vehicle is a polyhydric alcohol, and the various components are homogeneously blended into a single cohesive mass, the method comprising: incorporating 0.5% to 45% of at least one absorbent in the adhesive composition, the at least one absorbent being carboxymethyl cellulose, whereby the extent and/or rate of release of the at least one active agent is increased. In certain versions of the present invention, the extent of release and/or the rate of release of an active from a composition already including an absorbent can be increased by incorporating additional amounts of absorbent into the composition.

In another aspect, the present invention provides an adhesive composition comprising 30% to 80% of an adhesive, 0.5% to 45% of at least one absorbent, 0.5% to 10% of at least one active agent, 0.5% to 20% of at least one soluble crystallization inhibitor, and 0.5% to 20% of a vehicle, wherein the at least one active agent exists in a stable amorphous state within the adhesive composition, the adhesive is a styrene-isoprene hot melt adhesive, the at least one absorbent is carboxymethyl cellulose, the at least one active agent is an anti-rheumatic selected from the group consisting of diclofenac, ibuprofen, piroxicam, ketoprofen, thiocolchicoside, and methotrexate, the at least one crystallization inhibitor is polyvinylpyrrolidone, the vehicle is a polyhydric alcohol, and the various components are homogeneously blended into a single cohesive mass.

The adhesive composition may comprise 30% to 80% of a hot melt adhesive as the adhesive, 0.5% to 45% of the at least one absorbent, 0.5% to 10% of the at least one active agent, 0.5% to 20% of the polyvinylpyrrolidone as the at least one crystallization inhibitor, and 0.5% to 20% of at least one polyhydric alcohol as the vehicle.

In yet another aspect, the present invention provides an article adapted to be adhered to biological skin and release at least one active agent to the skin, the article defining a surface and comprising the adhesive composition of the present invention disposed on at least a portion of the surface.

**EP 2 968 201 B2**

In the article adapted to be adhered to biological skin and release at least one active agent to the skin, the adhesive composition may comprise 30% to 80% of a hot melt adhesive as the adhesive, 0.5% to 45% of the at least one absorbent, 0.5% to 10% of the at least one active agent, 0.5% to 20% of the polyvinylpyrrolidone as the at least one crystallization inhibitor, and 0.5% to 20% of at least one polyhydric alcohol as the vehicle.

The adhesive composition of the present invention may be obtained by a method of forming the adhesive composition which releases at least one active agent. The method comprises providing an adhesive component including the adhesive and the vehicle. The method also comprises incorporating 0.5% to 45% of the at least one absorbent, 0.5% to 10% of the at least one active agent, and 0.5% to 20% of the polyvinylpyrrolidone as the at least one crystallization inhibitor into the adhesive component to thereby form the adhesive composition which releases the at least one active agent.

BRIEF DESCRIPTION OF THE DRAWINGS

Figure 1 is a graph of solubility of an active agent as a function of a ratio of two components in compositions according to an embodiment of the present invention.
Figure 2 is a graph of flux of an active agent from various compositions described in greater detail herein, several of which are in accordance with embodiments of the present invention.
Figure 3 is a graph of recovery of an active agent from several compositions described in greater detail herein and in accordance with embodiments of the present invention.
Figures 4 and 5 are graphs of elution of an active agent from several compositions described in greater detail herein and in accordance with embodiments of the present invention.

In many applications, it is desirable to incorporate a drug or other active agents into an adhesive matrix to combine the therapeutic effects of the drug or active ingredient(s) with the cushioning, moisture-absorbing, and protective properties of the adhesive. The present invention relates to incorporating one or more active agents into an adhesive and particularly into a hydrocolloid adhesive. As described herein, it has been discovered that incorporation of one or more absorbents and/or absorbent agents in an adhesive composition in particular proportions and/or in conjunction with certain other agents, improves release and/or characteristics thereof of one or more actives, active agents, drugs, and/or pharmaceuticals from the adhesive composition.

Several aspects of the present invention, periodically referred to herein as a "drug-in-adhesive" system, include, but are not limited to the following. The active agents are controllably released from the adhesive composition into a receiving medium such as for example adjacent or underlying tissue. The active agents exist in a stable, amorphous state, i.e. noncrystalline state, within the adhesive composition. Crystallization of the active agents can cause unpredictable variations in the rate of active agent release or drug delivery, and therefore, such instability is to be avoided. The adhesive compositions exhibit good adhesive properties so that the compositions can remain on the skin for an extended period of time, for example 24 hours or more.

Generally, the present invention provides for certain combinations of adhesive, absorbent, drug or active agent, crystallization inhibitor, and vehicle to provide the noted aspects. The resulting adhesive compositions provide a drug-release feature without compromising absorption or adhesion performance. The active agent utilized herein is an anti-rheumatic selected from the group consisting of diclofenac, ibuprofen, piroxicam, ketoprofen, thiocolchicoside, and methotrexate.

The present invention provides an extensive range of adhesive compositions which can be tailored to controllably release the active agent from the adhesive composition. Table 1 set forth below lists exemplary adhesive compositions and their components. Table 1 is not in accordance with the present invention. All percentages expressed herein are percentages by weight, unless noted otherwise.

Table 1 - Adhesive Compositions

| Weight Percentage | Component |
|---|---|
| 20% to 90% | Adhesive |
| 0.1% to 50% | Absorbent |
| 0.1% to 20% | Active Agent(s) |
| 0.1% to 30% | Crystallization Inhibitor |
| 0.1% to 30% | Vehicle |

3

**Adhesive(s)**

The adhesive or adhesive component utilized herein is a styrene-isoprene hot melt adhesive. In certain aspects, the compositions of the present invention may comprise a hot melt pressure sensitive adhesive.

Thermoplastic elastomers such as styrene-isoprene-styrene block copolymers may be used, and these may require optional tackifiers and plasticisers. Blends or mixtures of elastomers may be more easily employed.

Particularly suitable as bases for the pressure sensitive adhesives of the present invention are rubbers such as linear or radial A-B-A block copolymers or mixtures of these A-B-A block copolymers with simple A-B block copolymers. These block copolymers are based on styrene-isoprene.

Suitable styrene-isoprene copolymers for the practice of the present invention are exemplified by a blend of linear styrene/isoprene/styrene triblock copolymer and linear styrene/isoprene diblock copolymer. Such a material is available from Shell Chemical as Kraton D-1161 and has a bound styrene content of about 15% and a diblock content of 17%. A second example is a blend of linear styrene/isoprene/styrene triblock copolymer and linear styrene/isoprene diblock copolymer available from Shell Chemical as Kraton D-1117 and which has a bound styrene content of about 17% and a diblock content of 33%.

An example of a suitable hydrogenated styrene-diene copolymer is a thermoplastic elastomer comprising a blend of clear linear triblock and diblock copolymer based on styrene and ethylene/butylene, with a bound styrene of 14% mass. Such a material is commercially available from Shell Chemical Company as Kraton G-1657. Another example is Kraton G-1652 from Shell Chemical Company, which is a thermoplastic elastomer comprised of a clear linear triblock copolymer based on styrene and ethylene-butylene, S-E/B-S, with a bound styrene content of about 30% by weight. Also suitable are polymers in which there is a combination of chemically saturated blocks and chemically unsaturated blocks. For example, a branched copolymer consisting of two polyisoprene chains attached to the rubber midblock of a styrene/ethylene-butylene/styrene triblock copolymer may be suitable. Such a material is available from Shell Chemical Company as Kraton Research Product RP6919. This material has a styrene content of 18%, an isoprene content of 36% and an ethylene-butylene content of 46% by weight. Also, a low styrene synthetic copolymer of butadiene and styrene, commonly called SBR rubber, can be used as a solid rubber.

Also particularly suitable are acrylic pressure sensitive adhesives, exemplified by an acrylic hot melt adhesive manufactured by Schenectedy Chemicals and having the designation Durotac 401. Another example is an acrylic solvent adhesive from Avery Chemicals called Polytex 7600.

**Absorbent(s)**

Herein, carboxymethyl cellulose is utilized as the absorbent. Generally, the absorbent includes one or more hydrophilic polymers that are soluble or insoluble but swellable in water, as the moisture-absorbing component. Suitable insoluble swellable polymers include crosslinked sodium carboxymethyl cellulose and crystalline sodium carboxymethyl cellulose. Suitable water soluble polymers include sodium carboxymethyl cellulose.

The super absorbent polymer (SAP), if used in the adhesive compositions, comprises a water-swellable, hydrogel-forming absorbent polymer capable of absorbing large quantities of liquids such as water, body fluids (e.g., urine, blood), and the like. Additionally, the SAP is capable of retaining such absorbed fluids under moderate pressures. Typically the SAP absorbs many times its own weight in water, preferably at least 50 times, more preferably at least 100 times, most preferably at least 150 times its weight in water. Additionally, the SAP exhibits good saline fluid absorption under load and high saline fluid absorption capacity. Typically the SAP absorbs at least 10 times, preferably at least 30 times, more preferably at least 50 times its weight in saline fluid. Even though the SAP is capable of absorbing many times its own weight in water and/or saline, it does not dissolve in these fluids.

The ability of the SAP to absorb water and/or saline fluid is related to the degree of crosslinking present in the SAP. Increasing the degree of crosslinking increases the SAP's total fluid holding capacity under load. The degree of crosslinking is preferably optimized to obtain a composition in which the rate and amount of absorbency are optimized. Preferred SAPs are at least 10%, more preferably from 10% to 50%, and most preferably from 20% to 40% crosslinked.

Super absorbent polymers useful in the present invention include crosslinked products of carboxymethyl cellulose polymers.

The super absorbent polymer(s) is typically in the form of particles and preferably are spherical and have an average particle size of from 1 micrometer ($\mu$m) to 400 ($\mu$m). Preferably the particles have an average particle size of from 20 $\mu$m to 200 $\mu$m, and more preferably from 20 $\mu$m to 150 $\mu$m. In one embodiment, the particle size of the particles is less than 150 $\mu$m, or less than 100 $\mu$m.

Thus in summary, the absorbent(s) utilized in the adhesive compositions of the present invention is typically one or more agents selected from (i) insoluble swellable polymers, (ii) hydratable polymers, (iii) water soluble polymers, (iv) synthetic absorbents, (v) super absorbent polymers, and/or (vi) combinations of any one or more of (i)-(v).

As already mentioned above, carboxymethyl cellulose is utilized as the absorbent. For certain embodiments, more than one type or grade of carboxymethyl cellulose (CMC) may be utilized in the compositions and methods of the present invention. CMC is a cellulose ether comprised of repeating cellobiose units. These are composed of two anhydroglucose units (beta-glucopyranose residues). A parameter used in referring to grades of CMC is the degree of polymerization. This is the number of anhydroglucose units which are joined through 1,4-glucosidic linkages. Each anhydroglucose unit contains three hydroxyl groups. By substituting carboxymethyl groups for some of the hydrogens of the hydroxyl groups, sodium carboxymethyl cellulose is obtained. The average number of hydroxyl groups substituted per anhydroglucose unit is known as the "degree of substitution". If all three hydroxyls are replaced, the maximum theoretical degree of substitution is 3.0 (impossible to practice).

Another parameter used in reference to CMC is average chain length or degree of polymerization. Average chain length (or the degree of polymerization) and the previously noted degree of substitution determine molecular weight of the CMC polymer.

For many embodiments, the CMC utilized in the present invention has a degree of substitution of from 0.2 to 1.5, and in other embodiments from 0.7 to 1.2. In particular embodiments, the degree of substitution of the CMC is from 0.65 to 0.90. The molecular weight of CMC is typically within a range of from 17,000 to 700,000. The present invention includes CMC grades having molecular weights less than 17,000 and greater than 700,000.

In certain versions of the present invention, a particularly useful absorbent is sodium carboxymethyl cellulose commercially available from various sources such as from Ashland Chemical under the designation AQUASORB A500.

**Active Agent(s)**

Generally, the active agent utilized herein is biologically active and can be incorporated within the adhesive composition in a stable manner or form. The active agent is soluble in polyhydric alcohol(s) which are utilized herein as the vehicle in the compositions. The active agent forms a complex with the crystallization inhibitor, described in greater detail herein, which is polyvinylpyrrolidone according to the present invention. The complex typically results from hydrogen bonding between the active and the inhibitor.

As already mentioned above, an anti-rheumatic selected from the group consisting of diclofenac, ibuprofen, piroxicam, ketoprofen, thiocolchicoside, and methotrexate is utilized herein as the active agent.

Crystallization Inhibitor(s)

The present invention makes use of one or more crystallization inhibitors in the compositions. Crystallization inhibitors are used to enhance the solubility and stability of the actives in the compositions. Crystallization inhibitors provide for higher concentrations/loading of active agents to be dissolved and once dissolved, inhibit the actives from subsequently precipitating out of solution. By use of these strategies, the crystallization inhibitors thereby increase the availability of the actives for application to human skin during transdermal drug delivery. Although not wishing to be bound to any particular theory, it is believed that, because more soluble actives are available within the adhesive matrix, the crystallization inhibitors indirectly increase the amount of actives delivered to the skin. High concentrations of dissolved active ingredients in the matrix of transdermal therapeutic systems generally make possible a high flow of active ingredients to and through the skin to aid in treatment.

Although a wide range of crystallization inhibitors can be incorporated to enhance the availability of the solvated actives for treatment, such as, for example, polyvinylpyrrolidone, polyacrylamides, polyvinyl alcohols, polyacrylic acids, caseins, gelatins, polyamines/polyethyleneimines, polyethylene glycols, cellulose, cellulose derivatives, methylcellulose, hydroxypropyl cellulose, ethyl cellulose, carboxymethylcellulose, non-urethane associated thickeners, quaternary ammonium alginates, xanthan, pectin, guar gum, guar gum derivatives, carrageenan, carboxypolymethylene, agar, polyethoxylated sorbitols, butyl methacrylate, butyl methacrylate derivatives, 2-dimethylaminoethyl methacrylate, methyl methacrylate, polyaminoamides, polyaminoimidazolines, polyetherurethaneamines, polyethylene oxide, polyacrylic acid, silica, silicon dioxide, starch, starch derivatives, dextrin, cyclodextrins, dextran, rosin esters, sterols, bile acids, polyglucosamines, monoacylglycerols, glycerol monooleate, glycerol monolinoleate, glycerol monopalmitate, glycerol monostearate, glycerol monolaurate, glycerol monocaprylate, glycerol monocaprate, and combinations or mixtures thereof, the at least one crystallization inhibitor is polyvinylpyrrolidone (PVP) according to the present invention.

PVP is a white, hygroscopic polymer with a weak characteristic odor. PVP is usually in powder form, although it can be in solution, and comprises the monomer N-vinylpyrrolidone as the base unit. By selecting suitable polymerization conditions, a wide range of molecular weights can be obtained, extending from low values of a few thousand daltons to approximately 2.2 million daltons, i.e. 2,200 kDa.

PVP can be either a homopolymer or copolymer, typically synthesized by free-radical polymerization in water or alcohols with a suitable initiator of vinylpyrrolidone (also referred to as N-vinylpyrrolidone, N-vinyl-2-pyrrolidone and N-vinyl-2-pyrrolidinone) as a monomeric unit. PVP polymers include soluble and insoluble homopolymeric PVPs, and copolymers such as vinylpyrrolidone/vinyl acetate and vinylpyrrolidone/dimethylamino-ethylmethacrylate. Substantially crosslinked homopolymers of PVP are insoluble and are generally known in the pharmaceutical industry under the designations polyvinylpolypyrrolidone, crospovidone and PVP. The copolymer vinylpyrrolidone-vinyl acetate is generally known in the pharmaceutical industry under the designations Copolyvidon(e), Copolyvidonum or VP-VAc.

The PVP included in the adhesive composition of the present invention is soluble. The term "soluble" when used with reference to PVP means that the polymer is soluble in water and generally is not substantially crosslinked, and has a weight average molecular weight of less than 2,200,000. In contrast to most polymers, soluble PVP is readily soluble in water and also in a large number of organic solvents, such as alcohols, amines, acids, chlorinated hydrocarbons, amides and lactams. Soluble PVP polymers have been identified in the pharmaceutical industry under a variety of names, the most commonly used include Povidone, Polyvidon(e), Polyvidonum, Polyvidonum, poly(N-vinyl-2-pyrrolidinone, poly(N-vinylbutyrolactam), poly(1-vinyl-2-pyrrolidone), poly[1-(2-oxo-1-pyrrolidinyl)ethylene]. PVP homopolymer is generally insoluble in the common esters, ethers, hydrocarbons and ketones. When dry, soluble PVP homopolymer is a light flaky powder, which absorbs up to 40% of its weight in water.

The amount and type of PVP required in the embodiments typically depends on the quantity and type of drug present in the adhesive composition, as well as the type of adhesives. Typically, the PVP is present in an amount from 0.1% to 30% by weight of the weight of the total adhesive composition. The soluble PVP for certain versions of the present invention has a weight average molecular weight of less than 2,200 kilodaltons (kDa), more particularly less than 100 kDa, and most particularly less than 54 kDa. In certain versions, it is useful to employ PVP having a weight average molecular weight from 2,000 to 2,200,000 (i.e. 2 kDa to 2,200 kDa), more particularly from 5,000 to 100,000 (i.e. 5 kDa to 100 kDa), and most particularly 7,000 to 54,000 (i.e. 7 kDa to 54 kDa). In certain versions of the present invention it is useful to employ PVP having certain characteristics and/or properties. For example, in certain embodiments, the PVP has a weight average molecular weight (Mw) of from 9 to 850 kilodaltons (kDa), and a number average molecular weight (Mn) of from 2 to 200 kDa. In certain aspects, the PVP has a glass transition temperature of from 110 °C to 180 °C. And, in certain embodiments, the PVP has a K-value of from 15 to 82. It is also contemplated to utilize PVP exhibiting all of these characteristics.

As noted, the at least one crystallization inhibitor is polyvinylpyrrolidone (PVP). In contrast to most polymers, PVP is readily soluble in water and a large number of organic solvents, such as alcohols, amines, acids, chlorinated hydrocarbons, amides and lactams. The polymer is generally insoluble in the common esters, ethers, hydrocarbons and ketones.

PVP is typically synthesized by free-radical polymerization of N-vinylpyrrolidone in water or alcohols with a suitable initiator. By selecting suitable polymerization conditions, a wide range of molecular weights can be obtained, extending from low values of a few thousand daltons to approximately 2.2 million daltons.

In certain versions of the present invention, it is advantageous to utilize one or more commercially available grades of PVP, such as those under the tradename LUVITEC® from BASF Corporation. BASF offers a wide range of vinylpyrrolidone homopolymers with different molecular weights (K-values) under the name LUVITEC® K. The products are available as a powder or as aqueous solutions. Characteristic parameters of LUVITEC® K grades are listed in Table 2.

Table 2 - Representative Grades of PVP

| | K-value | Solids content in % | pH-value (10% solution) | Residual NVP content in ppm | Brookfield-RVT Viscosity in mPa•s at 23 °C |
|---|---|---|---|---|---|
| LUVITEC® K 17 powder | 15-19 | 95.0-100.0 | 3.0-7.0 | ≤ 100 | 80-180 (40/2/100) |
| LUVITEC® K 30 powder | 27-33 | 95.0-100.0 | 3.0-7.0 | ≤ 100 | 80-140 (30/1/50) |
| LUVITEC® K 80 powder | 74-82 | 95.0-100.0 | 5.0-8.0 | ≤ 100 | 2500-7000 (20/6/100) |
| LUVITEC® K 85 powder | 84-88 | 95.0-100.0 | 5.0-9.0 | ≤ 100 | 5000-20000 (20/6/50) |
| LUVITEC® K 90 powder | 88-92 | 95.0-100.0 | 5.0-9.0 | ≤ 100 | 10000-25000 (20/7/100) |
| LUVITEC® K 90 HM powder | 92-96 | 95.0-100.0 | 5.0-9.0 | ≤ 100 | 15000-30000 (20/7/100) |
| LUVITEC® K 30 solution approx. 30% | 27-33 | 29.0-31.0 | 4.0-8.0 | ≤ 100 | 80-140 (30/1/50) |
| LUVITEC® K 60 solution approx. 35% | 52-62 | 34.0-36.0 | 7.0-9.0 | ≤ 300 | 2000-20000 (35/6/50) |
| LUVITEC® K 85 CQ solution approx. 20% | 83-88 | 19.0-21.0 | 7.0-9.0 | ≤ 100 | 5000-15000 (20/6/50) |

(continued)

|  | K-value | Solids content in % | pH-value (10% solution) | Residual NVP content in ppm | Brookfield-RVT Viscosity in mPa·s at 23 °C |
|---|---|---|---|---|---|
| LUVITEC® K 90 CQ solution approx. 10% | 90-98 | 9.5-10.5 | 7.0-9.0 | ≤ 50 | 300-1500 (10/4/100) |
| LUVITEC® K 90 solution approx. 20% | 90-98 | 19.0-21.0 | 7.0-9.0 | ≤ 100 | 10000-40000 (20/7/100) |
| LUVITEC® K 115 CQ solution approx. 10% | 114-130 | 10.5-11.5 | 7.0-9.0 | ≤ 50 | 2000-5000 (11/6/100) |

Table 3 set forth below presents typical properties of various grades of PVP commercially available under the LUVITEC tradename.

Table 3 - Typical Properties of PVP

| LUVITEC® | K 17 | K 30 | K 60 | K 80 | K 85 | K 90 | K 90 HM | K 115 |
|---|---|---|---|---|---|---|---|---|
| Molecular weight (GPC): Mw in kDa | 9 | 50 | 450 | 850 | 1100 | 1400 | 1800 | 2200 |
| Mn in kDa | 2 | 14 | 140 | 200 | 250 | 325 | 375 | 400 |
| Ash content in %* | | | | ≤0.02 | | | | |
| Rel. Viscosity (1% in water, 23 °C capillary viscometer) | 1.09 (5%: 1.53) | 1.25 | 1.93 | 3.09 | 3.74 | 5.09 | 5.69 | 12.1 (0.1%: 1.33) |
| Glass transition temperature in °C (DSC) | 110 | 175 | 175 | 180 | 180 | 180 | 180 | 180 |
| Particle size in μm (Sympatec-Helos Rodos) | | | | | | | | |
| $X_{10}$% | 15 | 25 | Only available as solution | 60 | 90 | 90 | 90 | Only available as solution |
| $X_{50}$% | 25 | 75 | | 160 | 180 | 180 | 180 | |
| $X_{90}$% | 100 | 130 | | 320 | 350 | 350 | 350 | |
| Color (10% solution, according to Europ. Pharmacopoeia) | | | Brighter than BY5/B5/R6 | | | | | |
| Moisture absorption at saturation in % | | | 20 (50% rel. humidity, 23 °C) 40 (75% rel. humidity, 23 °C) | | | | | |

In certain versions of the present invention it is useful to employ PVP having certain characteristics and/or properties. For example, in certain embodiments, the PVP has a weight average molecular weight (Mw) of from 9 to 850 kDa, and a number average molecular weight (Mn) of from 2 to 200 kDa. In certain aspects, the PVP has a glass transition temperature of from 110 °C to 180 °C. And, in certain embodiments, the PVP has a K-value of from 15 to 82. It is also contemplated to utilize PVP exhibiting all of these characteristics.

**Vehicle(s)**

And, a wide array of vehicles, carriers, and/or solvents can be utilized in the compositions of the present invention. Herein, one or more polyhydric alcohols are utilized as the vehicle in the compositions of the present invention. Suitable examples of polyhydric alcohols include dihydric alcohols, such as ethylene glycols, polyethylene glycols, propylene glycols, 1,3- and 1,4-butanediols, 1,6-hexanediol, diethylene glycol, bis(hydroxymethyl)cyclohexane, bis(hydroxyethyl)benzene, hydrogenated bisphenol A, hydrogenated bisphenol F, polytetramethylene glycols, polyester diols and silanol-terminated polysiloxanes; trihydric alcohols, such as glycerol, trimethylol propane, trimethylol ethane, 1,2,3-butane triol, 1,2,6-hexane triol and polyester triols; and polyhydric alcohols having 4 to 8 or more hydroxyl groups, such as pentaerythritol, diglycerol, $\alpha$-methylglucoside, sorbitol, xylitol, mannitol, glucose, fructose, sucrose, and the like. However, it will be appreciated that the present invention includes the use of other carriers and/or solvents in addition to these polyhydric alcohols.

**Additional Aspects**

In certain embodiments, the adhesive composition is a hydrocolloid adhesive. A base hydrocolloid adhesive formulation generally comprises a hot melt adhesive blended with an absorbent such as sodium carboxymethylcellulose (CMC) or the like. Any of these formulations can be selected for the base adhesive in a drug-delivery application in accordance with the present invention.

For effective drug delivery it is advantageous to incorporate a polyhydric alcohol which acts primarily as a vehicle into which the drug is actually dissolved, but may also serve a secondary function as a skin penetration enhancer. Propylene glycol is an example of a polyhydric alcohol which serves both purposes. Other examples of polyhydric alcohol vehicles include glycerol and polyethylene glycols, typically with molecular weights between 200 and 1,000 Da, or any mixtures thereof.

According to the present invention, polyvinylpyrrolidone as a particular crystallization inhibitor is included to stabilize the drug or active agent(s). Polyvinylpyrrolidone (PVP) may include soluble PVP homopolymers of low molecular weight. PVP can also increase the viscosity of the polyol phase, in some cases creating a gel, to prevent the polyol phase from migrating and separating from the adhesive matrix.

Once blended together, these ingredients, e.g. certain polyhydric alcohols and certain PVP agents, form a complex multi-phase system in which the various components typically form separate domains but are nevertheless homogeneously blended into a single cohesive mass.

In view of these aspects, the present invention provides a hydrocolloid adhesive formulation as set forth below in Table 4. The active agent may for example be ibuprofen.

Table 4- Representative Adhesive Compositions

| Weight Percentage | Component |
|---|---|
| 30% to 80% | Hot Melt Adhesive |
| 0.5% to 45% | Absorbent |
| 0.5% to 10% | Active Agent(s) |
| 0.5% to 20% | Polyvinylpyrrolidone (PVP) (Crystallization Inhibitor) |
| 0.5% to 20% | Polyhydric Alcohol (Vehicle) |

In certain embodiments, the hot melt adhesive includes styrene-isoprene copolymers, the polyhydric alcohol is propylene glycol, the PVP is a soluble homopolymer with molecular weight less than 60 kDa, and the absorbent is sodium carboxymethyl cellulose (CMC).

Notably, these mixtures can be blended and extruded at temperatures less than 75 °C, which is the critical thermal breakdown temperature of many actives such as ibuprofen. This is a unique feature that cannot be achieved with solvent-based acrylic adhesives because of the high drying temperatures that such adhesives typically require. In certain applications, the adhesives can be prepared by incorporating the various components at temperatures in a range of 60 C to 70 °C.

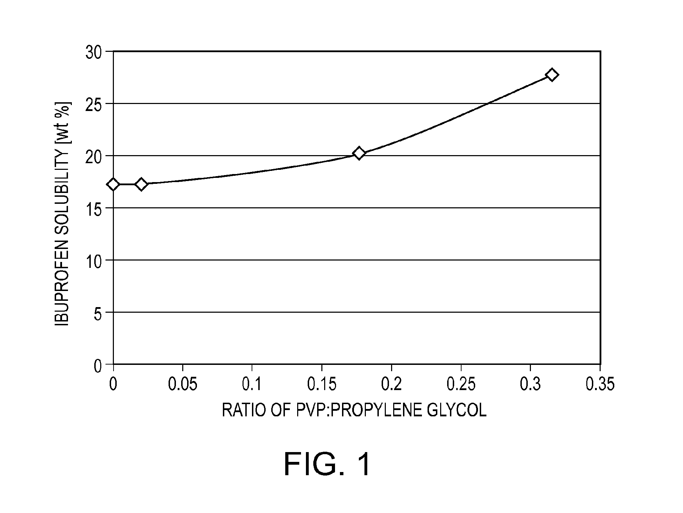

PVP enhances the solubility of certain actives such as ibuprofen in propylene glycol, as shown in Figure 1. Without PVP present, the room temperature saturation concentration is 17.3 wt % ibuprofen in propylene glycol. The solubility of ibuprofen increases to 27.7 wt % when the vehicle is changed to a mixture of 75% propylene glycol and 25% PVP (BASF LUVITEC K17, typical Mw of about 9,000 Da). The enhanced solubility enables increased loading of drug or other active(s) without risk of crystallization.

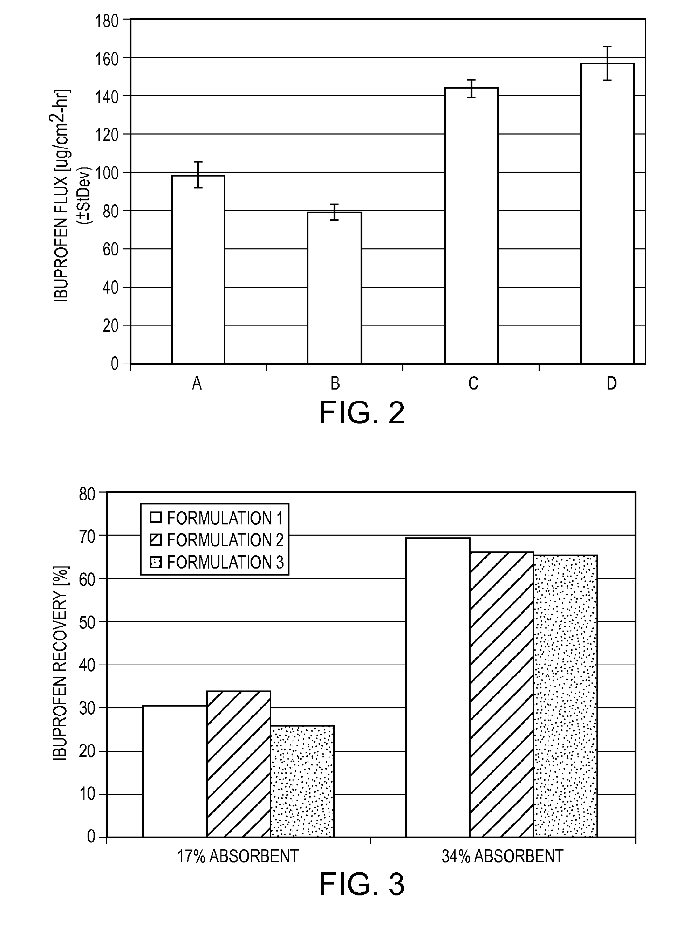

A mixture of propylene glycol and PVP also enhances the release kinetics of ibuprofen, as shown in Figure 2. Compared to a mixture of ibuprofen with hot melt adhesive alone, or compared to a mixture of ibuprofen with hot melt adhesive and absorbent; mixtures incorporating PVP/propylene glycol blends have approximately 50% higher release rates. Specifically, Figure 2 illustrates flux of ibuprofen released from various adhesive formulations measured using Franz diffusion cells employing a silicone rate-limiting membrane and 40% ethanol/saline as the receptor solution. In Figure 2, composition A is 5% ibuprofen in a hot melt adhesive. Composition B is 5% ibuprofen in a hot melt adhesive containing CMC. Composition C is 5% ibuprofen in a hot melt adhesive containing CMC and a mixture of 17% PVP in propylene glycol.

And, composition D is 5% ibuprofen in a hot melt adhesive containing CMC and a mixture of 43% PVP in propylene glycol. Compositions C and D represent embodiments of the present invention.

Formulations containing PVP are more stable in terms of ibuprofen crystallization when compared to non-PVP-containing counterparts. Differential scanning calorimetry of a 5% ibuprofen formulation containing 10% propylene glycol and 34% CMC reveals a thermal transition that is absent in an equivalent formulation containing PVP. The transition is attributed to the melting of crystals contained with this material that are absent in materials containing PVP.

In accordance with the present invention, adding one or more absorbents imparts an unexpected advantage beyond simply allowing the formulation to absorb fluid. Drug release is also enhanced by the inclusion of an absorbent material. In certain versions of the present invention, the extent of release of the one or more active agents is increased more than the increase in the amount of the absorbent. For example, in certain embodiments, the extent of release (or rate of release in certain versions) can be increased by 10% upon increasing the amount of absorbent in the adhesive composition by less than 10% such as for example 5%. This aspect may be expressed in terms of multiplication factors. For example, in certain versions of the present invention, upon increasing the amount of absorbent in the adhesive composition by a factor of 2.0 (or 200%), the extent of release of an active agent such as ibuprofen is increased by a factor greater than 2.0 (or greater than 200%).

Another particular advantage of the compositions of the present invention is that the compositions can be processed at low temperatures. This is particularly important for a drug such as ibuprofen, which degrades at 75 °C. In certain versions of the present invention, compositions can be processed at 65-70 °C. For a solvent-based system, for example, the resulting composition would need to be processed at much higher temperatures in order to dry it or remove vehicle or solvent. Similarly, rubber hot melts would be processed at temperatures of about 150 °C.

In addition to exhibiting the previously noted properties, in particular embodiments of the present invention, the adhesive compositions also exhibit a relatively high fluid handling characteristic or ability. The characteristic of relatively high fluid handling ability is exhibited in one or more fashions as follows.

In one aspect, the relatively high fluid handling ability of the adhesive compositions of the present invention is indicated by the compositions exhibiting a static absorption of at least 50 $g/m^2/24$ hours. In certain versions of the present invention, the adhesive compositions exhibit a static absorption of at least 100 $g/m^2/24$ hours; of at least 500 $g/m^2/24$ hours; of at least 1,000 $g/m^2/24$ hours; of at least 2,500 $g/m^2/24$ hours; and in certain embodiments of at least 5,000 $g/m^2/24$ hours. In certain versions, the adhesives exhibit a static absorption of from 5,000 to 10,000 $g/m^2/24$ hours.

In another aspect, the relatively high fluid handling characteristics of the adhesive compositions is indicated by its moisture vapor transmission rate (MVTR). Generally, the MVTR of the adhesive compositions of the present invention is at least 25 $g/m^2/24$ hours. In certain embodiments of the present invention, the adhesive compositions exhibit MVTR values of at least 50 $g/m^2/24$ hours; at least 100 $g/m^2/24$ hours; at least 200 $g/m^2/24$ hours; at least 350 $g/m^2/24$ hours; and in certain versions, greater than 500 $g/m^2/24$ hours. In certain versions, the adhesive compositions exhibit a MVTR from 500 to 1000 $g/m^2/24$ hours.

In certain versions of the present invention, the adhesive compositions exhibit a static absorption of at least 500 $g/m^2/24$ hours and an MVTR value of at least 25 $g/m^2/24$ hours. A description of determining static absorption and MVTR is provided herein.

## Methods

Disclosed herein is a method of forming an adhesive composition which releases at least one active agent. The method comprises providing an adhesive component including an adhesive and a vehicle. The adhesive and the vehicle are described herein. The method also comprises incorporating 0.5% to 45% of at least one absorbent, 0.5% to 10% of at least one active agent, and 0.5% to 20% of at least one crystallization inhibitor into the adhesive component to thereby form an adhesive composition which releases the at least one active agent. The absorbent(s), active agent(s), and inhibitor(s) are as described herein. The various components can be incorporated with one another, blended, and/or otherwise combined in techniques or operations known in the art so that the various components are homogeneously blended into a single cohesive mass.

The present invention also provides an adhesive composition for use as a medicament in a method of releasing at least one active agent to a region of interest such as for example on biological skin. The method comprises providing an article adapted for placement along biological skin. The article generally defines at least one face. The method also comprises providing an adhesive composition including an adhesive component, 0.5% to 45% of at least one absorbent, 0.5% to 10% of at least one active agent, 0.5% to 20% of a vehicle, and 0.5% to 20% of at least one crystallization inhibitor. The method further comprises depositing the adhesive composition onto the face of the article. And, the method comprises applying the article to a region of interest on biological skin, whereby the adhesive composition is directed toward and overlies the region of interest. The active agent(s) is then released from the adhesive composition and is transferred to the region of interest such as a wound or other region on biological skin. The components of the adhesive composition are as described herein.

In certain embodiments, the adhesive compositions of the present invention exhibit particular Peel on polyethylene values and/or particular Tack values. The measurement of these properties is described in greater detail herein in conjunction with various evaluations described herein. In certain versions, the adhesive compositions exhibit a Peel on polyethylene value of at least 0.197 N/cm (0.5 N/in), more particularly at least 0.591 N/cm (1.5 N/in), and more particularly at least 0.985 N/cm (2.5 N/in). In certain versions, the adhesive compositions exhibit a Tack value of at least 3.94 N/cm (10 N/in), more particularly at least 9.84 N/cm (25 N/in), and more particularly at least 15.7 N/cm (40 N/in).

Medical Articles

The adhesive compositions described herein can be used in association with a wide array of medical articles. Nonlimiting examples of such articles include wound dressings, surgical dressings, medical tapes, athletic tapes, surgical tapes, sensors, electrodes, ostomy appliances or related components such as sealing rings, catheters, connector fittings, catheter hubs, catheter adapters, fluid delivery tubes, electrical wires and cables, negative pressure wound therapy (NPWT) components, surgical drains, wound draining components, IV site dressings, prostheses, stoma pouches, buccal patches, transdermal patches, dentures, hairpieces, bandages, diapers, medical padding for example liposuction padding, hygiene pads, corn and callous pads, blister cushioning and protecting pads, toe cushioning pads, and pads for protecting and cushioning tube sites such as tracheotomy tubes. The medical articles include one or more regions or surfaces to which the adhesive compositions of the present invention are applied. Forming a layer, coating, or other region of adhesive on an article enables the article to be adhered to a wide range of surfaces, including skin. It will be understood that the present invention is not limited to any of these articles. Instead, the present invention includes the use of the adhesive compositions with other articles besides those noted herein. The medical articles may also include one or more layers covering the adhesive layer or coating such as a release liner.

Examples

Evaluations were undertaken to assess several adhesive compositions in accordance with the present invention. The materials used in the adhesive compositions were as follows.

Adhesive: T2650 (Avery Dennison), styrene-isoprene hot melt adhesive;

Absorbent: AQUASORB A500 (Ashland), sodium carboxymethyl cellulose;

PVP: LUVITEC K17 (BASF), soluble PVP homopolymer with average molecular weight of 9 kDa;

Drug: Ibuprofen (BASF), USP-grade;

Vehicle: propylene glycol.

Three different adhesive formulations were prepared as follows:

5% ibuprofen, 10% propylene glycol, 2% PVP (Formulation 1)

5% ibuprofen, 10% propylene glycol, 5% PVP (Formulation 2)

5% ibuprofen, 10% propylene glycol, 7.5% PVP (Formulation 3)

In a first set of trials, 17% absorbent was added (the remainder of the mixture being adhesive), and in a second set 34% absorbent was added. Samples were soaked in a mixture of ethanol and water for two hours and then the solvent was sampled to measure the total amount of ibuprofen extracted from the mixture. In all three cases, including 34% absorbent resulted in more than twice as much ibuprofen recovery, as shown in Figure 3. Specifically, as shown in Figure 3, the ibuprofen recovery for formulations 1 and 3 containing 17% absorbent was about 30% and 26%, respectively; and for the same formulations containing 34% absorbent, the ibuprofen recovery was increased to almost 70% and about 66%, respectively. These gains were greater than the relative increase in the proportion of absorbent, i.e. from 17% to 34%.

Thus, in certain aspects, the present invention provides methods of increasing the extent of release of one or more actives in an adhesive composition by increasing the amount or proportion of absorbent in the composition. In certain versions, increasing the amount of absorbent in the adhesive composition by a factor of 2.0 results in the extent of release of ibuprofen being increased by a factor greater than 2.0. Moreover, increasing the amount or proportion of absorbent also increases the rate and extent of ibuprofen release as detailed in the following evaluation.

In another evaluation, the effect of absorbent on ibuprofen release was investigated. In this evaluation, a series of formulations were prepared using T2560 (hot melt adhesive), AQUASORB A500 (sodium carboxymethyl cellulose absorbent), propylene glycol, and various grades of polyvinylpyrrolidone (LUVITEC K80, LUVITEC K30, and LUVITEC K17 from BASF). 5% ibuprofen was included in each formulation.

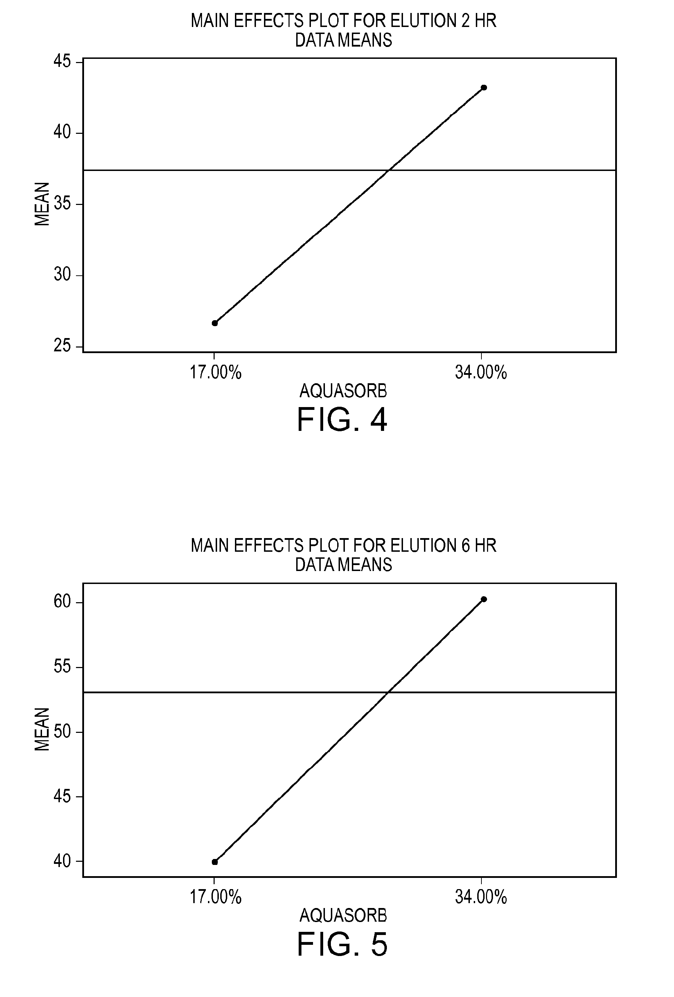

Ibuprofen elution was measured by soaking an amount or "patch" of adhesive in a mixture of 40% ethanol in saline (0.8% NaCl in water), and sampling the liquid phase after 2 hrs and again after 6 hrs. The concentration of ibuprofen in the solution was measured by HPLC and this value was used to calculate the ibuprofen released from the patch as a percentage of the total ibuprofen contained in the patch. The formulation list and results are set forth in Table 5 below. A Main Effects analysis using MINITAB® statistical software from Minitab Inc. was undertaken which revealed that higher amounts of A500 led to greater ibuprofen release. Referring to Figures 4 and 5, on average, doubling the amount of A500 from 17% to 34% led to a substantial increase in ibuprofen elution after both 2 and 6 hours of soak time.

Table 5 - Representative Formulations and Elution Results

| Formulation ID | T2560 Hot Melt Adhesive | A500 Absorbent | Propylene Glycol | LUVITEC K80 PVP | LUVITEC K30 PVP | LUVITEC K17 PVP | Ibuprofen | Elution 2 hrs [% of total ibu] | Elution 6 hrs [% of total ibu] |
|---|---|---|---|---|---|---|---|---|---|
| 1 | 66.00% | 17.00% | 10.0% | 2.0% | 0.00% | 0.00% | 5.00% | 20 | 30 |
| 2 | 49.00% | 34.00% | 10.0% | 2.0% | 0.00% | 0.00% | 5.00% | 56 | 79 |
| 3 | 63.00% | 17.00% | 10.0% | 5.0% | 0.00% | 0.00% | 5.00% | 32 | 29 |
| 4 | 46.00% | 34.00% | 10.0% | 5.0% | 0.00% | 0.00% | 5.00% | 31 | 59 |
| 5 | 60.50% | 17.00% | 10.0% | 7.5% | 0.00% | 0.00% | 5.00% | 17 | 25 |
| 6 | 43.50% | 34.00% | 10.0% | 7.5% | 0.00% | 0.00% | 5.00% | 20 | 39 |
| 7 | 66.00% | 17.00% | 10.0% | 0.0% | 0.00% | 2.00% | 5.00% | 31 | 52 |
| 8 | 49.00% | 34.00% | 10.0% | 0.0% | 0.00% | 2.00% | 5.00% | 69 | 69 |
| 9 | 63.00% | 17.00% | 10.0% | 0.0% | 0.00% | 5.00% | 5.00% | 34 | 63 |
| 10 | 46.00% | 34.00% | 10.0% | 0.0% | 0.00% | 5.00% | 5.00% | 66 | 75 |
| 11 | 60.50% | 17.00% | 10.0% | 0.0% | 0.00% | 7.50% | 5.00% | 26 | 42 |
| 12 | 43.50% | 34.00% | 10.0% | 0.0% | 0.00% | 7.50% | 5.00% | 66 | 71 |
| 13* | 61.00% | 34.00% | 0.0% | 0.0% | 0.00% | 0.00% | 5.00% | 33 | 50 |
| 14* | 51.00% | 34.00% | 10.0% | 0.0% | 0.00% | 0.00% | 5.00% | 37 | 55 |
| 15 | 49.00% | 34.00% | 10.0% | 0.0% | 2.00% | 0.00% | 5.00% | 37 | 62 |
| 16 | 46.00% | 34.00% | 10.0% | 0.0% | 5.00% | 0.00% | 5.00% | 32 | 56 |
| 17 | 43.50% | 34.00% | 10.0% | 0.0% | 7.50% | 0.00% | 5.00% | 30 | 50 |
| * not in accordance with the present invention | | | | | | | | | |

In another evaluation, various compositions were prepared. For each composition, a comparative composition was prepared.

For each resulting set of compositions, the following measurements were made: (i) adhesion to polyethylene, i.e. "Peel", (ii) Tack, (iii) release of active after 2 hours, i.e. "Elution 2 hr", (iv) release of active after 6 hours, i.e. "Elution 6 hr", and (v) fluid handling capacity, i.e. "FHC".

Specifically, samples were prepared and evaluated as follows.

Sample Preparation

Adhesive components were blended together in a sigma-blade mixer heated to approximately 70 °C. After blending, all adhesive specimens were pressed into sheets approximately 0.8 mm thick and laminated on one side to an adhesive-coated polyurethane film, 70 microns in thickness.

Peel

Specimens measuring one inch wide by approximately five inches long were applied to the surface of a polyethylene film using a 2.04 kg (4.5 lb) roller and allowed to dwell for approximately one minute before peeling off at a ninety degree angle and a rate of 30.5 cm/minute (12 inches/minute) using a tensile tester to record the average force required for removal.

Tack

Specimens were mounted to a fixture, adhesive side up. A piece of 0.127 mm (5 mil) polyester film measuring 15 cm long by 2.54 cm wide was formed into a loop and brought into contact with the adhesive surface, making a 2.54 cm (1 inch) $\times$ 2.54 cm (1 inch) contact area. Then the polyester loop was pulled away from the adhesive. A tensile tester was used to contact and pull the loop away from the adhesive at a crosshead speed of 30.5 cm/minute (12 inches/min), and the peak force measured during removal was taken as the quantitative measurement of tack.

Elution

A disk was cut from each specimen measuring 1.91 cm (0.75 inches) in diameter and placed in a vial containing approximately 20 mL of a solution of ethanol and saline (40% ethanol by volume in a solution of 0.9% sodium chloride in water by weight). The vial was tumbled in ambient temperature conditions, and after two and six hours samples of the solution were taken out for analysis. The concentration of ibuprofen in the solution was quantified using ultraviolet absorbance allowing the percentage ibuprofen release to be calculated as a percentage by weight of the total amount present in the sample.

Fluid Handling Capacity

Fluid Handling Capacity is a measure of the combined ability of the composite to take up moisture and to evaporate it to the environment. This test is performed by laminating a sample cut to the size of a Paddington cup to the cup on the side having the rubber ring. The circular sealing ring is placed on the sample of the cup and the screws are secured. The cup is weighed (W1). The cup is then turned upside down and filled with 20 ml of a saline solution (0.9% wt NaCl and 0.04% wt $CaCl_2$ in deionized water). The metal sealing place is secured to the top side of the cup. The filled cup is weighed (W2). The cup is placed sample side down into an oven at 37 °C for 24 hours. After 24 hours, the cup is removed from the oven and allowed to cool to room temperature for 30 minutes. The cup is then weighed (W3). The metal sealing plate is removed and the cup is emptied. The cup is allowed to stand for 15 minutes on a tissue to remove the saline solution, and then weighed (W4). The test conditions are 23 °C ($\pm$2 °C) and 50% ($\pm$2%) relative humidity. The samples tested herein had a thickness of 0.8 mm. The Moisture Vapor Transmission Rate (MVTR) equals (W2-W3)$\times$1000. The Static Absorption equals (W4-W1)$\times$1000. The Fluid Handling Capacity (FHC) in g/10 cm$^2$/24 hours is determined as follows:

$$FHC=(W2-W3)+(W4-W1)$$

Tables 6, 8, 10, and 12 present Comparative Composition A-D, respectively, lacking absorbent. These tables also present compositions in accordance with the present invention in which absorbent is included, i.e. Compositions 1 and 3. Compositions 2 and 4 are not in accordance with the present invention, lacking polyvinylpyrrolidone. Tables 7, 9, 11, and 13 present the results of testing in which Peel, Tack, elution, and fluid handling capacity were evaluated.

Table 6 - Formulations

| | Composition 1 | Comparative Composition A |
|---|---|---|
| | Parts [Weight Basis] | Parts [Weight Basis] |
| Hot Melt Adhesive | 100 | 100 |
| Propylene Glycol | 4.4 | 4.3 |
| Polyvinylpyrrolidone | 2.9 | 2.7 |
| Ibuprofen | 7.2 | 6.9 |
| Absorbent | 28 | 0 |

Table 7 - Results of Testing

| | Composition 1 | Comparative Composition A |
|---|---|---|
| Peel [N/cm] ([N/in]) | 1.10 (2.8) | 0.197 (0.5) |
| Tack [N/in] | 50 | 40 |
| Elution 2 hr [%] | 20 | 12 |
| Elution 6 hr [%] | 35 | 23 |
| FHC [g/m$^2$/day] | 6,300 | 200 |

Table 8 - Formulations

| | Composition 2 | Comparative Composition B |
|---|---|---|
| | Parts [Weight Basis] | Parts [Weight Basis] |
| Hot Melt Adhesive | 100 | 100 |
| Propylene Glycol | 4.3 | 4.5 |
| Polyvinylpyrrolidone | 0 | 0 |
| Ibuprofen | 7.2 | 7.4 |
| Absorbent | 29 | 0 |

Table 9 - Results of Testing

| | Composition 2 | Comparative Composition B |
|---|---|---|
| Peel [N/cm] ([N/in]) | 0.0 | 0.276 (0.7) |
| Tack [N/in] | 10 | 35 |
| Elution 2 hr [%] | 22 | 15 |
| Elution 6 hr [%] | 44 | 28 |
| FHC [g/m$^2$/day] | 7,500 | 200 |

Table 10 - Formulations

| | Composition 3 | Comparative Composition C |
|---|---|---|
| | Parts [Weight Basis] | Parts [Weight Basis] |
| Hot Melt Adhesive | 100 | 100 |
| Propylene Glycol | 6.9 | 7.3 |
| Polyvinylpyrrolidone | 8.9 | 9.4 |
| Ibuprofen | 8.3 | 8.7 |

(continued)

| | Composition 3 | Comparative Composition C |
|---|---|---|
| | Parts [Weight Basis] | Parts [Weight Basis] |
| Absorbent | 38 | 0 |

Table 11 - Results of Testing

| | Composition 3 | Comparative Composition C |
|---|---|---|
| Peel [N/cm] ([N/in]) | 1.02 (2.6) | 0.118 (0.3) |
| Tack [N/in] | 41 | 42 |
| Elution 2 hr [%] | 16 | 9 |
| Elution 6 hr [%] | 31 | 19 |
| FHC [g/m$^2$/day] | 8,400 | 300 |

Table 12 - Formulations

| | Composition 4 | Comparative Composition D |
|---|---|---|
| | Parts [Weight Basis] | Parts [Weight Basis] |
| Hot Melt Adhesive | 100 | 100 |
| Propylene Glycol | 7.0 | 7.0 |
| Polyvinylpyrrolidone | 0 | 0 |
| Ibuprofen | 8.2 | 8.3 |
| Absorbent | 38 | 0 |

Table 13 - Results of Testing

| | Composition 4 | Comparative Composition D |
|---|---|---|
| Peel [N/cm] ([N/in]) | 0.0 | 0.0394 (0.1) |
| Tack [N/in] | 8 | 38 |
| Elution 2 hr [%] | 25 | 15 |
| Elution 6 hr [%] | 52 | 28 |
| FHC [g/m$^2$/day] | 8,300 | 200 |

As evident from Tables 6-13, incorporating absorbent into an adhesive formulation significantly increases elution of ibuprofen (both after 2 hours and after 6 hours). Specifically, as shown in Tables 6 and 7, incorporating absorbent into an adhesive composition resulted in an increase in elution at 2 hours from 12% to 20%, and an increase in elution at 6 hours from 23% to 35%. As shown in Tables 8 and 9, incorporating absorbent into an adhesive composition resulted in an increase in elution at 2 hours from 15% to 22%, and an increase in elution at 6 hours from 28% to 44%. As shown in Tables 10 and 11, incorporating absorbent into an adhesive composition resulted in an increase in elution at 2 hours from 9% to 16%, and an increase in elution at 6 hours from 19% to 31%. And, as shown in Tables 12 and 13, incorporating absorbent into an adhesive composition resulted in an increase in elution at 2 hours from 15% to 25%, and an increase in elution at 6 hours from 28% to 52%.

Claims

1.  An adhesive composition comprising:

    30% to 80% of an adhesive;

0.5% to 45% of at least one absorbent;
0.5% to 10% of at least one active agent;
0.5% to 20% of at least one soluble crystallization inhibitor; and
0.5% to 20% of a vehicle,
wherein the at least one active agent exists in a stable amorphous state within the adhesive composition, the adhesive is a styrene-isoprene hot melt adhesive, the at least one absorbent is carboxymethyl cellulose, the at least one active agent is an anti-rheumatic selected from the group consisting of diclofenac, ibuprofen, piroxicam, ketoprofen, thiocolchicoside, and methotrexate, the at least one crystallization inhibitor is polyvinylpyrrolidone, the vehicle is a polyhydric alcohol, and the various components are homogeneously blended into a single cohesive mass.

2. The adhesive composition of claim 1, wherein the polyhydric alcohol is selected from the group consisting of propylene glycols, glycerols, polyethylene glycols, and combinations thereof.

3. The adhesive composition of claim 1, wherein the adhesive composition exhibits a Peel on polyethylene value of at least 0.197 N/cm (0.5 N/in), preferably at least 0.591 N/cm (1.5 N/in).

4. The adhesive composition of claim 1, wherein the adhesive composition exhibits a static absorption of at least 50 g/m$^2$/24 hours.

5. An article adapted to be adhered to biological skin and release at least one active agent to the skin, the article defining a surface and comprising the adhesive composition of claim 1 disposed on at least a portion of the surface.

6. An adhesive composition for use as a medicament wherein the method of releasing at least one active agent to a region of interest on biological skin comprises:

providing an article adapted for placement along biological skin, the article defining at least one face;
providing an adhesive composition including 30% to 80% of an adhesive component, 0.5% to 45% of at least one absorbent, 0.5% to 10% of at least one active agent, 0.5% to 20% of a vehicle, and 0.5% to 20% of at least one crystallization inhibitor, wherein the at least one active agent exists in a stable amorphous state within the adhesive composition, the adhesive component is a styrene-isoprene hot melt adhesive, the at least one absorbent is carboxymethyl cellulose, the at least one active agent is an anti-rheumatic selected from the group consisting of diclofenac, ibuprofen, piroxicam, ketoprofen, thiocolchicoside, and methotrexate, the at least one crystallization inhibitor is polyvinylpyrrolidone, the vehicle is a polyhydric alcohol, and the various components are homogeneously blended into a single cohesive mass;
depositing the adhesive composition onto the face of the article; and
applying the article to a region of interest on biological skin, whereby the adhesive composition is directed toward and overlies the region of interest.

7. The composition for use according to claim 6, wherein the adhesive composition contacts at least a portion of the region of interest on biological skin.

8. The composition for use according to claim 6, wherein the at least one active agent is ibuprofen.

9. The composition for use according to claim 6, wherein the polyhydric alcohol is selected from the group consisting of propylene glycol, glycerol, polyethylene glycol, and combinations thereof, preferably wherein the polyhydric alcohol is propylene glycol.

10. The composition for use according to claim 6, wherein the adhesive composition exhibits a Peel on polyethylene value of at least 0.197 N/cm (0.5 N/in), preferably at least 0.591 N/cm (1.5 N/in).

11. The composition for use according to claim 6, wherein the adhesive composition exhibits a static absorption of at least 50 g/m$^2$/24 hours.

12. An adhesive composition for use as a medicament wherein the increase of at least one of (i) extent of release and (ii) rate of release of at least one active agent from the adhesive composition including 30% to 80% of an adhesive component, 0.5% to 10% of at least one active agent, 0.5% to 20% of a vehicle, and 0.5% to 20% of at least one crystallization inhibitor, wherein the at least one active agent exists in a stable amorphous state within the adhesive composition, the adhesive component is a styrene-isoprene hot melt adhesive, the at least one active agent is an anti-rheumatic selected from the group consisting of diclofenac, ibuprofen, piroxicam, ketoprofen, thiocolchicoside, and methotrexate, the at least one crystallization inhibitor is polyvinylpyrrolidone, the vehicle is a polyhydric alcohol, and the various components are homogeneously blended into a single cohesive mass, the method comprising: incorporating 0.5% to 45% of at least one absorbent in the adhesive composition, the at least one absorbent being carboxymethyl cellulose, whereby the extent and/or rate of release of the at least one active agent is increased.

13. The composition for use according to claim 12, wherein the at least one active agent is ibuprofen.

14. The composition for use according to claim 12, wherein upon increasing the amount of absorbent in the adhesive composition by a factor of 2.0, the extent of release of ibuprofen is increased by a factor greater than 2.0.

15. The composition for use according to claim 12, wherein the adhesive composition exhibits a Peel on polyethylene value of at least 0.197 N/cm (0.5 N/in), preferably at least 0.591 N/cm (1.5 N/in).

**Patentansprüche**

1. Haftmittelzusammensetzung, umfassend:

   30 % bis 80 % eines Haftmittels;
   0,5 % bis 45 % mindestens eines Absorptionsmittels;
   0,5 % bis 10 % mindestens eines Wirkstoffs;
   0,5 % bis 20 % mindestens eines löslichen Kristallisationsinhibitors; und
   0,5 % bis 20 % eines Vehikels,
   wobei der mindestens eine Wirkstoff in einem stabilen amorphen Zustand innerhalb der Haftmittelzusammensetzung vorliegt, das Haftmittel ein Styrol-Isopren-Heißschmelzhaftmittel ist, das mindestens eine Absorptionsmittel Carboxymethylcellulose ist, der mindestens eine Wirkstoff ein Antirheumatikum, ausgewählt aus der Gruppe, bestehend aus Diclofenac, Ibuprofen, Piroxicam, Ketoprofen, Thiocolchicosid und Methotrexat, ist, der mindestens eine Kristallisationsinhibitor Polyvinylpyrrolidon ist, das Vehikel ein mehrwertiger Alkohol ist und die verschiedenen Komponenten homogen zu einer einzigen kohäsiven Masse vermischt sind.

2. Haftmittelzusammensetzung nach Anspruch 1, wobei der mehrwertige Alkohol aus der Gruppe, bestehend aus Propylenglykolen, Glycerinen, Polyethylenglykolen und Kombinationen davon, ausgewählt ist.

3. Haftmittelzusammensetzung nach Anspruch 1, wobei die Haftmittelzusammensetzung einen Schälwert auf Polyethylen von mindestens 0,197 N/cm (0,5 N/in), vorzugsweise mindestens 0,591 N/cm (1,5 N/in), aufweist.

4. Haftmittelzusammensetzung nach Anspruch 1, wobei die Haftmittelzusammensetzung eine statische Absorption von mindestens 50 g/m$^2$/24 Stunden aufweist.

5. Artikel, der geeignet ist, an biologischer Haut geklebt zu werden und mindestens einen Wirkstoff an die Haut freizusetzen, wobei der Artikel eine Oberfläche definiert und die Haftmittelzusammensetzung nach Anspruch 1 umfasst, die auf mindestens einem Teil der Oberfläche angeordnet ist.

6. Haftmittelzusammensetzung zur Verwendung als ein Medikament, wobei das Verfahren zum Freisetzen mindestens eines Wirkstoffs an einen interessierenden Bereich auf biologischer Haut umfasst:

   Bereitstellen eines Artikels, der zur Anordnung entlang biologischer Haut geeignet ist, wobei der Artikel mindestens eine Fläche definiert;
   Bereitstellen einer Haftmittelzusammensetzung, die 30 % bis 80 % einer Haftmittelkomponente, 0,5 % bis 45 % mindestens eines Absorptionsmittels, 0,5 % bis 10 % mindestens eines Wirkstoffs, 0,5 % bis 20 % eines Vehikels und 0,5 % bis 20 % mindestens eines Kristallisationsinhibitors einschließt, wobei der mindestens eine Wirkstoff in einem stabilen amorphen Zustand innerhalb der Haftmittelzusammensetzung vorliegt, die Haftmittelkomponente ein Styrol-Isopren-Heißschmelzhaftmittel ist, das mindestens eine Absorptionsmittel Carboxymethylcellulose ist, der mindestens eine Wirkstoff ein Antirheumatikum, ausgewählt aus der Gruppe, bestehend aus Diclofenac, Ibuprofen, Piroxicam, Ketoprofen, Thiocolchicosid und Methotrexat, ist, der mindestens eine Kristallisationsinhibitor Polyvinylpyrrolidon ist, das Vehikel ein mehrwertiger Alkohol ist und die verschiedenen Komponenten homogen zu einer einzigen kohäsiven Masse vermischt sind; Auftragen der Haftmittelzusammensetzung auf die Fläche des Artikels; und

Auftragen des Artikels auf einen interessierenden Bereich auf biologischer Haut, wobei die Haftmittelzusammensetzung auf den interessierenden Bereich gerichtet ist und diesen bedeckt.

**7.** Haftmittelzusammensetzung zur Verwendung nach Anspruch 6, wobei die Haftmittelzusammensetzung mindestens einen Teil des interessierenden Bereichs auf biologischer Haut berührt.

**8.** Haftmittelzusammensetzung zur Verwendung nach Anspruch 6, wobei der mindestens eine Wirkstoff Ibuprofen ist.

**9.** Haftmittelzusammensetzung zur Verwendung nach Anspruch 6, wobei der mehrwertige Alkohol aus der Gruppe, bestehend aus Propylenglykol, Glycerin, Polyethylenglykol und Kombinationen davon, ausgewählt ist, wobei der mehrwertige Alkohol vorzugsweise Propylenglykol ist.

**10.** Haftmittelzusammensetzung zur Verwendung nach Anspruch 6, wobei die Haftmittelzusammensetzung einen Schälwert auf Polyethylen von mindestens 0,197 N/cm (0,5 N/in), vorzugsweise mindestens 0,591 N/cm (1,5 N/in), aufweist.

**11.** Haftmittelzusammensetzung zur Verwendung nach Anspruch 6, wobei die Haftmittelzusammensetzung eine statische Absorption von mindestens 50 g/m$^2$/24 Stunden aufweist.

**12.** Haftmittelzusammensetzung zur Verwendung als ein Medikament, wobei der Anstieg von mindestens einem von (i) Ausmaß der Freisetzung und (ii) Freisetzungsrate von mindestens einem Wirkstoff aus der Haftmittelzusammensetzung, die 30 % bis 80 % einer Haftmittelkomponente, 0,5 % bis 10 % mindestens eines Wirkstoffs, 0,5 % bis 20 % eines Vehikels und 0,5 % bis 20 % mindestens eines Kristallisationsinhibitors einschließt, wobei der mindestens eine Wirkstoff in einem stabilen amorphen Zustand innerhalb der Haftmittelzusammensetzung vorliegt, die Haftmittelkomponente ein Styrol-Isopren-Heißschmelzhaftmittel ist, das mindestens eine Absorptionsmittel Carboxymethylcellulose ist, der mindestens eine Wirkstoff ein Antirheumatikum, ausgewählt aus der Gruppe, bestehend aus Diclofenac, Ibuprofen, Piroxicam, Ketoprofen, Thiocolchicosid und Methotrexat, ist, der mindestens eine Kristallisationsinhibitor Polyvinylpyrrolidon ist, das Vehikel ein mehrwertiger Alkohol ist und die verschiedenen Komponenten homogen zu einer einzigen kohäsiven Masse vermischt sind, wobei das Verfahren umfasst: Einarbeiten mindestens eines Absorptionsmittels in die Haftmittelzusammensetzung, wodurch das Ausmaß und/oder die Rate der Freisetzung des mindestens einen Wirkstoffs erhöht wird.

**13.** Haftmittelzusammensetzung zur Verwendung nach Anspruch 12, wobei der mindestens eine Wirkstoff Ibuprofen ist.

**14.** Haftmittelzusammensetzung zur Verwendung nach Anspruch 12, wobei bei Erhöhung der Menge an Absorptionsmittel in der Haftmittelzusammensetzung um einen Faktor von 2,0 das Ausmaß der Freisetzung von Ibuprofen um einen Faktor von mehr als 2,0 erhöht wird.

**15.** Haftmittelzusammensetzung zur Verwendung nach Anspruch 12, wobei die Haftmittelzusammensetzung einen Schälwert auf Polyethylen von mindestens 0,197 N/cm (0,5 N/in), vorzugsweise mindestens 0,591 N/cm (1,5 N/in), aufweist.

## Revendications

**1.** Composition adhésive comprenant :

30 % à 80 % d'un adhésif ;
0,5 % à 45 % d'au moins un absorbant ;
0,5 % à 10 % d'au moins un agent actif ;
0,5 % à 20 % d'au moins un inhibiteur de cristallisation soluble ; et
0,5 % à 20 % d'un véhicule,
dans laquelle l'au moins un agent actif existe dans un état amorphe stable au sein de la composition adhésive, l'adhésif est un adhésif thermofusible de styrène-isoprène, l'au moins un absorbant est la carboxyméthylcellulose, l'au moins un agent actif est un antirhumatismal sélectionné parmi le groupe constitué du diclofénac, de l'ibuprofène, du piroxicam, du kétoprofène, du thiocolchicoside et du méthotrexate, l'au moins un inhibiteur de cristallisation est la polyvinylpyrrolidone, le véhicule est un alcool polyhydrique, et les divers composants sont mélangés de manière homogène en une masse cohésive unique.

2. Composition adhésive selon la revendication 1, dans laquelle l'alcool polyhydrique est sélectionné parmi le groupe constitué de propylèneglycols, glycérols, polyéthylèneglycols et de combinaisons de ceux-ci.

3. Composition adhésive selon la revendication 1, dans laquelle la composition adhésive présente une valeur de pelage sur polyéthylène d'au moins 0,197 N/cm (0,5 N/pouce), de préférence d'au moins 0,591 N/cm (1,5 N/pouce).

4. Composition adhésive selon la revendication 1, dans laquelle la composition adhésive présente une absorption statique d'au moins 50 g/m$^2$/24 heures.

5. Article adapté à être collé à de la peau biologique et libérer au moins un agent actif vers la peau, l'article définissant une surface et comprenant la composition adhésive selon la revendication 1 disposée sur au moins une portion de la surface.

6. Composition adhésive destinée à être utilisée en tant que médicament, dans laquelle le procédé de libération d'au moins un agent actif vers une région pertinente sur de la peau biologique comprend :

   fournir un article adapté au placement le long de peau biologique, l'article définissant au moins une face ; fournir une composition adhésive incluant 30 % à 80 % d'un composant adhésif, 0,5 % à 45 % d'au moins un absorbant, 0,5 % à 10 % d'au moins un agent actif, 0,5 % à 20 % d'un véhicule et 0,5 % à 20 % d'au moins un inhibiteur de cristallisation, dans laquelle l'au moins un agent actif existe dans un état amorphe stable au sein de la composition adhésive, le composant adhésif est un adhésif thermofusible de styrène-isoprène, l'au moins un absorbant est la carboxyméthylcellulose, l'au moins un agent actif est un antirhumatismal sélectionné parmi le groupe constitué du diclofénac, de l'ibuprofène, du piroxicam, du kétoprofène, du thiocolchicoside et du méthotrexate, l'au moins un inhibiteur de cristallisation est la polyvinylpyrrolidone, le véhicule est un alcool polyhydrique et les divers composants sont mélangés de manière homogène en une masse cohésive unique ; déposer la composition adhésive sur la face de l'article ; et appliquer l'article à une région pertinente sur de la peau biologique, moyennant quoi la composition adhésive est dirigée vers et recouvre la région pertinente.

7. Composition destinée à être utilisée selon la revendication 6, dans laquelle la composition adhésive vient en contact avec au moins une portion de la région pertinente sur de la peau biologique.

8. Composition destinée à être utilisée selon la revendication 6, dans laquelle l'au moins un agent actif est l'ibuprofène.

9. Composition destinée à être utilisée selon la revendication 6, dans laquelle l'alcool polyhydrique est sélectionné parmi le groupe constitué du propylèneglycol, glycérol, polyéthylèneglycol et de combinaisons de ceux-ci, de préférence dans laquelle l'alcool polyhydrique est le propylèneglycol.

10. Composition destinée à être utilisée selon la revendication 6, dans laquelle la composition adhésive présente une valeur de pelage sur polyéthylène d'au moins 0,197 N/cm (0,5 N/pouce), de préférence d'au moins 0,591 N/cm (1,5 N/pouce).

11. Composition destinée à être utilisée selon la revendication 6, dans laquelle la composition adhésive présente une absorption statique d'au moins 50 g/m$^2$/24 heures.

12. Composition adhésive destinée à être utilisée en tant que médicament, dans laquelle l'augmentation d'au moins une de (i) l'étendue de libération et (ii) la vitesse de libération d'au moins un agent actif de la composition adhésive incluant 30 % à 80 % d'un composant adhésif, 0,5 % à 10 % d'au moins un agent actif, 0,5 % à 20 % d'un véhicule et 0,5 % à 20 % d'au moins un inhibiteur de cristallisation, dans laquelle l'au moins un agent actif existe dans un état amorphe stable au sein de la composition adhésive, le composant adhésif est un adhésif thermofusible de styrène-isoprène, l'au moins un agent actif est un antirhumatismal sélectionné parmi le groupe constitué du diclofénac, de l'ibuprofène, du piroxicam, du kétoprofène, du thiocolchicoside et du méthotrexate, l'au moins un inhibiteur de cristallisation est la polyvinylpyrrolidone, le véhicule est un alcool polyhydrique, et les divers composants sont mélangés de manière homogène en une masse cohésive unique, le procédé comprenant : incorporer 0,5 % à 45 % d'au moins un absorbant dans la composition adhésive, l'au moins un absorbant étant la carboxyméthylcellulose, moyennant quoi l'étendue et/ou la vitesse de libération de l'au moins un agent actif est augmentée.

13. Composition destinée à être utilisée selon la revendication 12, dans laquelle l'au moins un agent actif est l'ibuprofène.

14. Composition destinée à être utilisée selon la revendication 12, dans laquelle dès l'augmentation de la quantité d'absorbant dans la composition adhésive d'un facteur de 2,0, l'étendue de libération d'ibuprofène est augmentée d'un facteur supérieur à 2,0.

15. Composition destinée à être utilisée selon la revendication 12, dans laquelle la composition adhésive présente une valeur de pelage sur polyéthylène d'au moins 0,197 N/cm (0,5 N/pouce), de préférence d'au moins 0,591 N/cm (1,5 N/pouce).

FIG. 1

FIG. 2

FIG. 3

MAIN EFFECTS PLOT FOR ELUTION 2 HR
DATA MEANS

FIG. 4

MAIN EFFECTS PLOT FOR ELUTION 6 HR
DATA MEANS

FIG. 5

**EP 2 968 201 B2**

**REFERENCES CITED IN THE DESCRIPTION**

**Patent documents cited in the description**

- EP 2110125 A1